# EUROPEAN PATENT SPECIFICATION

(11) **EP 3 049 560 B1**
(45) Date of publication and mention of the grant of the patent: **12.05.2021**
(21) Application number: 14848662.4
(22) Date of filing: 22.09.2014
(51) Int. Cl.: C40B 40/14, B32B 9/04, C08J 7/18

(54) **METHODS OF USING DETECTABLE ARRAYS FOR DIAGNOSIS**
VERWENDUNG VON NACHWEIS-ARRAYS ZUR DIAGNOSE
UTILISATION RÉSEAUX DÉTECTABLES POUR LA DIAGNOSTIC

(30) Priority: 24.09.2013 US 201361881754 P
(43) Date of publication of application: 03.08.2016
(73) Proprietor: Entopsis, LLC, Medley, FL 33166 (US)
(72) Inventor: PILOTO, Obdulio, Coral Gables Florida 33134 (US); CHEONG, Ian Shen-Yi, Finksburg Maryland 21048 (US)
(74) Representative: Schiweck Weinzierl Koch Patentanwälte Partnerschaft mbB
(86) International application number: PCT/US2014/056822
(87) International publication number: WO 2015/047958

(56) References cited:
- WO-A2-01/94946
- WO-A2-03/079402
- KR-B1- 100 713 786
- US-A1- 2003 108 949
- US-A1- 2003 218 130
- US-A1- 2010 200 405
- US-A1- 2012 220 491
- US-B1- 6 306 598
- US-B2- 7 326 776
- Bridget Wilson ET AL: "Monitoring Proteins and Protein Networks Using Reverse Phase Protein Arrays", Disease markers, 1 January 2010 (2010-01-01), pages 225-232, XP055587895, United States DOI: 10.3233/DMA-2010-0705 Retrieved from the Internet: URL:https://www.ncbi.nlm.nih.gov/pmc/artic les/PMC3833243/pdf/DM28-04-240248.pdf

## Description

### CROSS REFERENCE TO RELATED APPLICATIONS

This application claims the benefit under 35 U.S.C. § 119(e) of U.S. Provisional Application No. 61/881,754, filed September 24, 2013.

### BACKGROUND

Detectable arrays can bind or immobilize an analyte on a solid support for detecting the presence, absence, or concentration of the analyte. Typically, detectable arrays are limited to selectively binding a single analyte or a single type of analyte. For example, a detectable array may contain a plurality of DNA sequence probes for binding to DNA having sequences complementary to the sequences of the probes. In another example, arrays can include a plurality of potential ligand receptors for selectively binding to a ligand of interest.

The application of such prior-art arrays is limited. For example, because environmental samples may contain multiple analytes of interest, more than one array may be needed to bind and detect the presence of analytes. Further, while known arrays are specific for one analyte or one type of analyte, disease states of subjects may be characterized by the presence or absence of more than one analyte. Thus, there is a need for a detectable array that can both bind and detect multiple analytes, as well as methods of diagnosing using such detectable arrays.

WO 03/079402 A2 provides an adsorbent chip, which includes three components, a substrate, an intermediate layer of linker arms and an adsorbent film, which is attached to the linker arms. The adsorbent film is made up of a plurality of adsorbent particles, each of which includes a binding functionality. The disclosure also provides a method of making the chips of the disclosure in which the substrate-intermediate film cassette is formed and the adsorbent film is subsequently immobilized thereon. When the adsorbent film is from the same preparation across a particular batch of chips, the chips provide for the acquisition of data that are highly reproducible from one chip to the next throughout the particular batch of chips. Additionally, the disclosure provides methods for using the chips to perform assays.

US 2012/220491 A1 relates to analytes in a sample which are resolved by retentate chromatography in a procedure involving adsorbing the analytes on a substrate under a plurality of different selectivity conditions, and detecting the analytes retained on the substrate by desorption spectrometry. The methods are useful in biology and medicine, including clinical diagnostics and drug discovery.

WO 01/94946 A2 provides peptidomimetic protein-binding arrays, their manufacture, use, and application. The protein-binding array elements of the disclosure include a peptidomimetic segment linked to a solid support via a stable anchor. The disclosure contemplates peptidomimetic array element library synthesis, distribution, and spotting of array elements onto solid planar substrates, labeling of complex protein mixtures, and the analysis of differential protein binding to the array. The disclosure also enables the enrichment or purification, and subsequent sequencing or structural analysis of proteins that are identified as differential by the array screen. Kits including proteomic microarrays in accordance with the disclosure are also provided.

KR 100 713 786 B1 provides a probe and a method of making the probe that is removably insertable into a gas phase ion spectrometer, the probe comprising a substrate having a surface and a hydrogel material on the surface, the hydrogel material comprising binding functionalities for binding with an analyte detectable by the gas phase ion spectrometer. The disclosure also provides a probe and a method of making the probe that is removably insertable into a gas phase ion spectrometer, the probe comprising a substrate having a surface and a plurality of particles that are uniform in diameter on the surface, the particles comprising binding functionalities for binding with an analyte detectable by the gas phase ion spectrometer. Further, the disclosure provides a system comprising the probe of the present invention and a gas phase ion spectrometer comprising an energy source that directs light to the probe surface to desorb an analyte and a detector in communication with the probe surface that detects the desorbed analyte. The disclosure also provides a method for desorbing an analyte from a probe surface, the method comprising exposing the binding functionalities to a sample containing an analyte under conditions to allow binding between the analyte and the binding functionalities, and desorbing the analyte from the probe by gas phase ion spectrometry.

US 2003/218130 A1 provides a substrate having a polymerized, polysaccharide-based hydrogel attached to the surface. The hydrogel can be derivatized with binding functionalities that bind analytes from a sample. The disclosure further provides methods of using the device and gels that are capable of selectively binding one or more analytes from a sample.

The scientific publication of B. Wilson et al. in Disease Markers 28 (2010), pages 225-232 relates to monitoring protein and protein networks using reverse phase protein arrays.

### SUMMARY OF INVENTION

The present invention relates to a label-free method of detecting an unlabeled analyte on a detectable array,
said detectable array comprising:
a substrate with a plurality of surfaces for binding one or more analytes, each surface independently comprising:
one or more substrate coatings on the surface for fixing one or more macromolecules to the surface of the substrate, said one or more substrate coatings comprising at least one silane or at least one siloxane; and
one or more macromolecules affixed to at least a portion of the one or more substrate coatings, the one or more macromolecules being arranged in a pattern on the substrate coating and comprising a plurality of binding sites for binding a plurality of analytes;
wherein each macromolecule has been affixed to the substrate coating by polymerizing a functional group on the silane or siloxane into the macromolecule backbone;
wherein each macromolecule comprises a polymer of monomers selected from a group consisting of acrylamide; 2-carboxyethyl acrylate; acrylic acid; N-[tris(hydroxymethyl)methy] acrylamide; hydroxypropyl acrylates; 4-hydroxybutyl acrylate; N-hydroxyethyl acrylamide; N,N-dimethylacrylamide; N-(1,1-dimethyl-3-oxobutyl) acrylamide; N-iso-propylacrylamide; (meth)acrylates; 2-cyanoethyl acrylate; ethylene glycol phenyl ether acrylate; N-tert-octylacrylamide; 1-(acryloyloxy)-3-(methacryloyloxy)-2-propanol; bis-acrylamide; trimethylolpropane triacrylate, and combinations thereof; and
wherein the identity, pattern, or both identity and pattern of the one or more macromolecules on the one or more substrate coatings of each of the plurality of surfaces is not identical to the identity, pattern, or both identity and pattern of the one or more macromolecules on any other substrate coating of any other of the plurality of surfaces;
said label-free method comprising:
contacting the unlabeled analyte with the detectable array to affix at least a portion of the analyte to the detectable array; and
heating the detectable array with the unlabeled analyte affixed thereto to cause a color change of at least one of the analyte and the detectable array, wherein the heating induces a non-enzymatic browning reaction, and wherein the heating comprises heating at a temperature of about 120°C to about 300°C for about 1 minute to about 5 minutes; and
assessing the color of each of the plurality of surfaces of the substrate by colorimetric analytical technique.

In some embodiments the non-enzymatic browning reaction is at least one of a Maillard reaction and caramelization.

In some embodiments the one or more substrate coatings comprise one or more acrylosiloxanes.

In some embodiments the one or more substrate coatings comprise one or more of 3-methacryloxypropyl trimethoxy silane, 3-acryloxypropyl trimethoxy silane, N-(3-acryloxy-2-hydroxypropyl-3-aminopropyltriethoxysilane, and 3-methacryloxypropyl dimethylchlorosilane.

In some embodiments the substrate comprises at least 2 chemically distinct macromolecules, and each chemically distinct macromolecule is affixed to a different surface of the plurality of surfaces of the substrate. Optionally, the one or more macromolecules comprise at least 12 chemically distinct macromolecules, at least 72 chemically distinct macromolecules, at least 96 chemically distinct macromolecules, or at least 288 chemically distinct macromolecules.

In some embodiments the substrate comprises one or more of glass, plastic, metal, composites, acrylics, or biologically active substrates.

In some embodiments the unlabeled analyte is selected from (i) a body sample of a subject, (ii) an *in-vitro* sample, (iii) an environmental sample, and (iv) a component of one of the foregoing.

In some embodiments the unlabeled analyte is at least one component of blood, serum, plasma, urine, stool, saliva, bile, spinal fluid, interstitial fluid, gastric juice, tears, solvent, and milk.

In some embodiments the unlabeled analyte comprises a protein or peptide.

Embodiments of the invention are also disclosed throughout the present description and Figures, and are the subject-matter of the appended claims.

Disclosed herein within the context of the method of the invention is a detectable array which can comprise a substrate with a plurality of surface for binding one or more analytes, each surface independently comprising one or more substrate coatings for fixing one or more macromolecules to the surface of the substrate and one or more macromolecules affixed to at least a portion of the one or more substrate coatings, the one or more macromolecules being arranged in a pattern on the substrate coating and comprising a plurality of unbiased binding sites for binding a plurality of analytes, wherein the identity, pattern, or both identity and pattern of the one or more macromolecules on the one or more substrate coatings on each of the plurality of surfaces is not identical to the identity, pattern, or both identity and pattern of the one or more macromolecules on any other substrate coating of any other of the plurality of surfaces and wherein the presence or absence of one or more analytes bound to each of the plurality of surfaces is detectable by a plurality of detection methods.

According to the invention, the one or more substrate coatings comprises at least one silane or at least one siloxane. For example, the silanes or siloxanes may be acrylosiloxanes, particularly one or more of 3-methacryloxypropyl trimethoxy silane, 3-acryloxypropyl trimethoxy silane, N-(3-acryloxy-2-hydroxypropyl-3-aminopropyltriethoxysilane, and 3-methacryloxy propyldimethylchlorosilane. Also disclosed herein within the context of the invention is that the one or more macromolecules may comprise one or more of polymers, surfactants, nanospheres, nanotubes, dendrimers, microspheres, and polymerized microspheres, for example, one or more of (meth)acrylamides, (meth)acrylates, glycerol, and N,N'(alkylene)bisacrylamide. In another embodiment, the one or more macromolecules comprise at least one copolymer. In another embodiment, the one or more macromolecules comprise one or more monomers, or two or more monomers, selected from the group consisting of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemethyl-3-oxobutyl)acrylamide, N-isopropylacrylamide, ethylene glycol phenyl ether acrylate, N,N'-methylenebisacrylamide, 1,1,3,3,3-Hexafluoroisopropyl acrylate, and N-*tert*-octylacrylamide. In a further embodiment, the one or more macromolecules may comprise one or more cross-linkers, such as at least one of bis-acrylamide, trimethylolpropane triacrylate, bisphenol A-bis(2-hydrxypropyl)acrylate, and 1-(acryloyloxy)-3-(methacryloyloxy)-3-methacryloyloxy)-2-propanol. In a further embodiment, the one or more macromolecules comprise a plurality of chemically distinct macromolecules, wherein each chemically distinct macromolecule is affixed to a different surface of the plurality of surfaces of the array. In additional embodiments, the one or macromolecules comprise at least 2, at least 12, at least 72, at least 96, or at least 288 chemically distinct macromolecules.

The plurality of detection methods may include one or more of Maillard reaction, caramelizing reaction with one or more amine reactive dyes, reaction with one or more thiol reactive dyes, reaction with one or more cellular dyes, reaction with one or more solvatochromic dyes, reaction with one or more acid indicators, reaction with one or more base indicators, reaction with one or more labeled antibodies, luminescence, surface texture analysis, photo-scanning, microscopy, photo-scanning with reflectance or transmittance illumination, photography with reflectance or transmittance illumination, mass spectrometry and spectroscopy. According to the invention, detection methods include a non-enzymatic browning reaction.

In an additional embodiment, the substrate is glass.

In an additional embodiment, the substrate comprises one or more of glass, plastic, metal, composites, acrylics, or biologically active substrates, e.g., wood.

In another additional embodiment, one or more analytes are bound to the one or more macromolecules. In a further additional embodiment, the detectable array further comprises one or more small molecules, proteins, peptides, nucleotides, nucleosides, bacteria, viruses, fungi cells, animal cells, or yeast cells bound to the one or more macromolecules.

Also disclosed herein within the context of the invention is a system for diagnosing a disease, disorder or condition can comprise an input element for receiving one or more images of a first detectable array or a representation thereof, wherein the first detectable array is any of the aforementioned first detectable arrays, a database comprising one or more images of a plurality of second detectable arrays or representations thereof, wherein the second detectable array is any of the aforementioned first detectable arrays, and a comparison element for comparing the one or more images of the first detectable array or a representation thereof with the one or more images of a plurality of second detectable arrays or representations thereof. Each of the images of the plurality of second detectable arrays or representations thereof may be associated with a subject, an in-vitro sample, or an environmental sample having a known disease, disorder, or condition. The first detectable array or representation thereof may be associated with a subject, in-vitro sample, or condition having an unknown disease, disorder, or condition state. The comparison element of the system may be capable of predicting a disease state of a subject, an in-vitro sample, or an environmental sample having an unknown disease state. The one or more images of the first detectable array or representation thereof may be false color images. The one or more images of the first detectable array or representation thereof may represent one or more of fluorescence, phosphorescence, texture, roughness, color, ultraviolet absorption, infrared absorption, or lack of one or more of the foregoing, of the plurality of surfaces of the substrate.

In an embodiment, a method of determining disease, disorder, or condition state in a subject, in-vitro sample, or environmental sample, by using a method according to the present invention, comprises contacting a first detectable array, such as any of the arrays disclosed herein, with one or more analytes associated with the subject, in-vitro sample, or environmental sample. In another embodiment, the contacting step comprises contacting the first detectable array with one or more body samples of the subject. In a further embodiment, the contacting step includes contacting at least one of blood, serum, plasma, urine, stool, saliva, bile, spinal fluid, interstitial fluid, gastric juice, tears, solvent, and milk of the subject, in-vitro sample, or environmental sample. In yet another embodiment, the contacting step comprises contacting the first detectable array with one or more of plasma and urine of the subject.

In a still further embodiment, the method further comprises obtaining the one or more analytes associated with the subject. In another further embodiment, the method further comprises detecting the one or more analytes associated with the subject, in-vitro sample, or environmental sample. In yet another embodiment, the method further comprises making one or more images of the detectable array or one or more representations thereof.

The method may comprises one or more of heating the detectable array, causing a Maillard reaction of at least one of the one or more analytes, caramelizing at least one of the one or more analytes, reacting at least of the one or more analytes with one or more amine reactive dyes, reacting at least one of the one or more analytes with one or more thiol reactive dyes, reacting at least of the one or more analytes with one or more solvatochromic dyes, reacting at least one of the one or more analytes with one or more cellular dyes, reacting at least one of the one or more analytes with one or more labeled antibodies, reacting at least of the one or more analytes with one or more acid indicators, reacting at least of the one or more analytes with one or more base indicators, detecting the luminescence or lack thereof of the detectable array, surface texture analysis, photo-scanning, microscopy, photo-scanning with reflectance or transmittance illumination, photography with reflectance or transmittance illumination, mass spectrometry and spectroscopy.

Also disclosed herein within the contexts of the invention is a method of making a detectable array, such as any of the detectable arrays described herein, which comprises independently contacting the plurality of surfaces with at least one substrate coating to form a plurality of independently coated surfaces, and independently affixing at least one macromolecule or one or more precursors thereof to each of the independently coated surfaces. The method may further comprise polymerizing at least one of the one or more precursors thereof on at least one of the independently coated surfaces. The method may include initiating polymerization of at least one of the at least one precursors by applying one or more of light, heat, or a chemical initiator to the one or more precursors. The method may comprise initiating polymerization of at least one of the one or more precursors by applying one or more of electromagnetic radiation (e.g., microwaves, ultraviolet light, visible light, heat), sound or a chemical initiator to the one or more precursors.

According to the invention, a label-free method of detecting an unlabeled analyte comprises contacting the unlabeled analyte with a detectable array to affix at least a portion of the analyte to the detectable array and heating the detectable array with unlabeled analyte affixed thereto to cause a color change of at least one of the analyte and the detectable array. In a further embodiment, the detectable array includes at least one array selected from the group consisting of an analytical microarray, a reverse-phase micro assay, a functional microarray, a cell-containing microarray, an expression microarray, and a high-throughput array. In a still further embodiment, the detectable array includes at least one array selected from the group consisting of an antibody array, and ELISA array, a peptide array, a protein array, a nucleotide array, a nucleoside array, an RNA array, a DNA array, a DNA-protein array, and a small molecule array. In a yet further embodiment, the unlabeled analyte is one or more of a body sample of a subject, an in-vitro sample, a environmental sample, and at least one component of one of the foregoing. In another further embodiment, the unlabeled analyte is at least one component of blood, serum, plasma, urine, stool, saliva, bile, spinal fluid, interstitial fluid, gastric juice, tears, solvent, and milk. According to the invention, the heating induces a non-enzymatic browning reaction. In yet another further embodiment, the non-enzymatic browning reaction is at least one of a Maillard reaction and caramelization. In another embodiment, heating comprises heating at a sufficient temperature and for a sufficient time to induce one or more of caramelization and a Maillard reaction. According to the invention, heating comprises heating at a temperature of about 120° C to about 300° C for about 1 minute to about 5 minutes.

### BRIEF DESCRIPTION OF THE DRAWINGS

Figure 1 is a schematic drawing of a system for diagnosing a subject, in-vitro sample, or environmental sample.
Figure 2 is a detectable array with bound proteins.
Figure 3 is a detectable array with bound yeast cells.
Figure 4 is a detectable array with bound small molecules.

### DETAILED DESCRIPTION

### Definitions

Unless otherwise defined herein, all terms used in this Application are to be afforded their usual meaning in the art, as they would be understood by a person of ordinary skill at the time of the invention. It should be understood that throughout this application singular forms, such as "a," "an," and "the" are often used for convenience; however, singular forms are intended to include the plural unless specifically limited to the singular either explicitly or by context.

"Analytes" are entities of interest that can bind to (either covalently, ionicaly, physically, or by any other means) and be detected on a detectable array. Analytes can include, but are not limited to, small molecules, such as vitamins and minerals, cells, proteins, peptides, and the like.

A "subject" can include any plant or animal, particularly animals, such as mammals, and most particularly humans.

"Macromolecules" include oligomers, polymers, dendimers, nanospheres, nanotubes, and the like.

"Polymers" includes both synthetic and naturally occurring polymers, such as homopolymers, copolymers, such as block copolymers, graft copolymers, alternating copolymers, and random copolymers, and also polypeptides, DNA, RNA, and the like.

"Small molecules" include biologically or environmentally relevant molecules having a molecular weight lower than that of macromolecules.

"Binding sites" are locations designed to bind one or more analytes. Binding sites are "unbiased" when they are not designed to be selective for a single analyte or a single type of analyte, and "biased" when they are designed to be selective for a single analyte or a single type of analyte.

"Diagnosis" refers to an estimation of the likelihood that a subject, in-vitro sample, or environmental sample has, does not have, or is susceptible to having at a future time, a particular disease, disorder, or condition state. A diagnosis can be quantitative, for example, expressed as a percentage or fractional likelihood, or qualitative.

A "cloud-computing environment" refers to one or more computers, computer servers, computer readable devices, and the like, containing data, software, files, or other computer-readable or computer-executable material that can be accessed by a plurality of remote devices. The remote devices can be computers, mobile telephones that are specially adapted to access, read, or execute software (e.g., smart-phone), tablet computers, and the like.

A detectable array can comprise a substrate with a plurality of surfaces for binding one or more analytes. The substrate can be made of any suitable substrate material, such as one or more of plastic, glass, and ceramic, but is typically glass such as silicate or borosilicate glass. The plurality of surfaces can have any appropriate shape or size, depending on the shape or size of the substrate. For example, when the substrate is a plate, such as a glass, plate, the plurality of surfaces can be wells in the plate. When the substrate is a slide, each of the plurality of surfaces can be a different location on the slide. When the substrate is a particle, such as a ceramic particle, the plurality of surfaces can be pore or openings in the particle. In one embodiment, the substrate comprises at least one of, or one or more of, glass, plastic, metal, composites, acrylics, or biologically active substrates

Each of the plurality of surfaces can independently comprise one or more substrate coatings for fixing one or more macromolecules to the surface of the substrate. The one or more substrate coatings can, independently, coat all or a portion of each of the plurality of surfaces. The substrate coatings can be any coatings that contain appropriate chemical groups for fixing one or more macromolecules to the surface of the substrate. Thus, the identity of the substrate coatings will depend on the identity of the one or more macromolecules to be affixed to the substrate. For example, if the one or more macromolecules include nanotubes, then the substrate coating can have a chemical moiety that binds to nanotubes. As another example, if the one or more macromolecules includes polymer, then the substrate coating can have one or more functional groups that can polymerize into the polymer backbone, such as olefin. Thus, the substrate coating can include, for example, at least one silane or at least one siloxane. Particular siloxanes include one or more acrylosiloxanes, such as one or more of 3-methacryloxypropyl trimethoxy silane, 3-acryloxypropyl trimethoxy silane, N-(3-acryloxy-2-hydroxypropyl-3-aminopropyltriethoxysilane, and 3-methacryloxy propyldimethylchlorosilane.

The one or more macromolecules can include any macromolecules, and particularly macromolecules that can provide one or more unbiased binding sites. Such macromolecules can have one or more free functional groups for binding analytes, such as carbonyls, amines, amides, carboxylic acids, esters, alcohols, and the like, however, this is not required unless otherwise specified. For example, nanotubes may not contain any free functional groups but can instead bind one or more analytes by virtue of their size and shape. Similarly, polymers with or without free functional groups can bind one or more analytes based not only on the nature of the functional groups (if present), but also based on the shape of the polymers and their interaction with the analytes in three dimensions.

The macromolecules can be, for example, at least one of polymer, surfactant, nanosphere, nanotube, dendrimer, microsphere, and polymerized microsphere. When the macromolecules include polymer, the polymer can be a homopolymer or copolymer, but is typically a copolymer. The macromolecules can comprise one or more of (meth)acrylamides, (meth)acrylates, and N,N'-(alkylene)bisacrylamide. For example, the macromolecules can comprise one or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-*tert*-octylacrylamide. As another example, the macromolecules can comprise two or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-*tert*-octylacrylamide.

The macromolecules can also comprise at least one cross-linker. The cross-linker can be any cross-linker known in the art. Cross-linkers can include, for example, one or more molecules containing two, three, four, or more olefins or acrylic functional groups, such as one or more of bis-acrylamide, trimethylolpropane triacrylate, bisphenol A-bis(2-hydrxypropyl)acrylate, and 1-(acryloyloxy)-3-(methacryloyloxy)-3-methacryloyloxy)-2-propanol.

The one or more macromolecules can be arranged such that the identity or pattern of the one or more macromolecules on any of the one or more substrate coatings of each of the plurality of surfaces is not identical to the identity or pattern of the one or more macromolecules on any other substrate coating of any other of the plurality of surfaces. Thus, each substrate coating of each of the plurality of surfaces can contain macromolecules that are unique in either chemical identity, pattern of physical disposition, or both, with respect to the other surfaces of the substrate. This can be accomplished in a number of ways. For example, lithographic techniques, which are well known in the art, can be used to create different patterns of macromolecules on the different surfaces. As another example, when the substrate is a plate, the macromolecules fixed to each well of the plate can have different chemical identities. As a further example, when the substrate is a slide, each chemically distinct macromolecule can be affixed to a different location on the slide; in this case each of the different locations on the slide is a different surface, such that the slide comprises a plurality of surfaces with chemically distinct macromolecules affixed thereto. In these or other manners, the one or more macromolecules can comprise a plurality of chemically distinct macromolecules, and each chemically distinct macromolecule can be affixed to a different surface of the plurality of surfaces of the substrate. For example, the one or more macromolecules can comprise at least two, at least twelve, at least seventy-two, at least ninety-six, or at least two-hundred and eighty-eight chemically distinct macromolecules, each of which can be affixed to a different surface of the plurality of surfaces of the substrate.

The detectable array can further comprise one or more analytes bound to the one or more macromolecules. The one or more analytes can comprise one or more of small molecules, proteins, peptides, nucleotides, nucleosides, bacteria, viruses, fungi cells, yeast cells, and animal cells bound to at least one of the one or more macromolecules. In particular, a plurality of different analytes can be bound to the one or more macromolecules. For example, if the detectable array is contacted with the blood of a subject, the one or more macromolecules can bind to cells, such as red blood cells, white blood cells, t-cells, and the like, and also bind to proteins and small molecules that are present in the blood. In particular, when each of the plurality of surfaces has a different affixed macromolecules, different types and quantities of analytes can bind to each of the plurality of surfaces, thereby creating a detectable pattern of bound analytes.

The presence or absence of one or more analytes bound to the one or more macromolecules can be detectable by a plurality of detection methods. The detection methods can also detect the one or more of absolute and relative amount of the one or more analytes and the identity of the one or more analytes, although this is not required unless otherwise specified. Any detection methods known in the art can be used. Exemplary detection methods include one or more of Maillard reaction, caramelizing, reaction with one or more amine reactive dyes, reaction with one or more thiol reactive dyes, reaction with one or more cellular dyes, reaction with one or more solvatochromic dyes, reaction with one or more acid indicators, reaction with one or more base indicators, reaction with one or more labeled antibodies, luminescence, surface texture analysis, photo-scanning, microscopy, photo-scanning with reflectance or transmittance illumination, photography with reflectance or transmittance illumination, mass spectrometry and spectroscopy.

The Maillard reaction is well known as the reaction that, for example, causes food to brown upon heating by reactions involving food components such as amino acids. A Maillard reaction can be used to detect analytes by heating the bound analytes, for example, to a temperature from about 200° C to about 400° C, such as about 275° C to about 325° C or about 300° C, for sufficient time to induce a Maillard reaction, such as from about 1 min to about 15 min, or about 5 min. The color of each of the plurality of surfaces of the substrate can then be assessed by colorimetric analytical techniques known in the art. For example, substrate can be scanned by a digital scanner to create an image of the plate's colors on a computer, which can then be analyzed by commercially available computer software.

Caramelizing is another well known reaction that, for example, can cause food to brown upon heating. Caramelizing involves different chemical processes than the Maillard reaction, such as pyrolysis of carbohydrates. Caramelizing can be used to detect analytes by heating the bound analytes, for example, to a temperature from about 100° C to about 210° C, such as about 130° C to about 210° C, or about 160° C to about 200° C, for a sufficient time to cause caramelization. The color of each of the plurality of surfaces of the substrate can then be assessed by colorimetric analytical techniques known in the art. For example, substrate can be scanned by a digital scanner to create an image of the plate's colors on a computer, which can then be analyzed by commercially available computer software.

Surface texture analysis can be performed, for example, using surface texture analyzers known in the art. The texture of the surface can be different depending on one or more of whether an analyte is bound to the surface, the nature or identity of the analyte bound to the surface, and the absolute or relative quantity of analyte bound to the surface.

Reaction with dyes or indicators, such as amine or thiol reactive dyes, cellular dyes, solvatochromic dyes, acid indicators or base indicators, can cause a color-change on one or more of the plurality of surfaces of the substrate. The nature and extent of the color change can depend on one or more of whether an analyte is bound to the surface, the nature or identity of the analyte bound to the surface, and the absolute or relative quantity of analyte bound to the surface. The color change can be analyzed by colorimetric analytical methods known in the art. For example, the plate can be scanned by a digital scanner to create an image of the plate's colors on a computer, which can then be analyzed by commercially available computer software.

Reaction with labeled antibodies can include reaction with any antibody that is labeled to be detectable when bound to an analyte. Such labeled antibodies are known in the art, and include fluorescence labeled antibodies, biotin/strepatvidin labeled antibodies, and the like.

Luminescence measurements can involve measuring, for example, the fluorescence or phosphorescence spectra of the plurality of surfaces of the substrate. The intensity, wavelength, and photo yield of the spectrum can depend on one or more of whether an analyte is bound to the surface, the nature or identity of the analyte bound to the surface, and the absolute or relative quantity of analyte bound to the surface.

Microscopy can include light microscopy, such as optical, ultraviolet, infrared, or luminescence microscopy. Microscopy can also include scanning techniques, such as scanning electron microscopy and atomic force microscopy.

In particular, Maillard reactions and caramelizing can have several advantages over other detection methods. For example, they can be label-free methods in that no labeling (*e.g.,* by addition of dye, stain, ligand, etc.) is required. Thus, staining with the Maillard reaction or caramelizing can avoid the use of expensive labeling dyes, stains, and the like, thereby reducing the cost of assays. Because these approaches require only a brief application of heat, rather than a complex chemical reaction or biological process, they are fast, easy to carry out, and less prone to user error than commercially available detection methods.

Detection by one or more of the plurality of detection methods can provide an image of the substrate or a representation thereof. The image can be a photograph or digital image. A representation thereof can include a false-color image where features such as texture, depth, surface roughness, luminescence intensity, and the like, are represented by colors or other indicia.

A system for diagnosing a disease, disorder, or condition, such as a disease of a subject, or a condition of an environmental sample, can include an input element for receiving one or more images of a first detectable array or a representation thereof. The input element can be any element that is used to both create and receive an image or representation thereof, such as a photo-scanner, or an element that is used solely to receive a digital representation of an image, such as a computer, web browser, cloud-computing environment, and the like.

The system can also include a database comprising one or more images of a plurality of second detectable arrays or representations thereof. The database can be stored physically, such as a photograph album containing images, but is more commonly stored digitally. For example the database can be stored on one or more computers, computer servers, computer-readable storage devices, such as hard drives, USB drives, and the like, or in a cloud-computing environment.

Each of the one or more images of the plurality of second detectable arrays or representations thereof can be associated with a subject, in-vitro sample, or environmental sample having a known disease, disorder, or condition state. For example, an image of the second detectable array can be associated with a subject having a known disease state, such as a bacterial infection or cancer; the subject can also be known to be disease-free. Similarly, an image or representation thereof can be associated with an in-vitro sample, such as a known in-vitro disease model, Petri dish with known components, and the like. An image or representation thereof can also be associated with an environmental sample having a known state, such as a wastewater sample of known composition, a soil sample with known contaminants, and the like.

The system can further include a comparison element for comparing the one or more images of the first detectable array or representation thereof to the one or more images of the plurality of second detectable arrays or representations thereof. The comparison element can be a simple physical element, such as one or more photograph albums comprising one or more images of a plurality of second detectable arrays or representations thereof for facilitating a visual comparison of the image of the first detectable array or representation thereof with the plurality of second detectable arrays or representations thereof. More commonly, the comparison element includes software that performs a series of steps to recognize features, such as color, size, location, and the like, and patterns of such features, of the image of the first detectable array or representation thereof, and compares those features to the one or more images of the plurality of second detectable arrays or representations thereof. The software can be executed on any suitable device, including a computer, mobile computer, mobile or stationary telephone equipped with software-executing capabilities (e.g., "smart-phone"), tablet computer, wearable computer, and the like. The software can also be executed in whole or in part from a computer server or a cloud-computing environment, which need not be in the same location as the input element. Thus, the comparison element can compare the one or more images of the first detectable array or representation thereof to the one or more images of the plurality of second detectable arrays or representations thereof by, for example, executing local software to perform this comparison or executing or causing to be executed software that is located in another location to perform this comparison. In some cases, the comparison element and the database can be the same device, although this is not required unless otherwise specified. Also, the comparison element need not comprise software; the comparison element can also be, for example, a photograph album or physical representation that facilitates comparison of the one or more images of the first detectable array or representation thereof to the one or more images of the plurality of second detectable arrays or representations thereof.

The comparison element can therefore be used to diagnose a disease, disorder, or condition, for example, of a subject, in-vitro sample, or environmental sample by comparing the one or more images of the first detectable array or representation thereof associated with the subject, in-vitro sample, or environmental sample to the one or more images of the plurality of second detectable arrays or representations thereof. The diagnosis can be achieved by comparing similarities and differences between the one or more images of the first detectable array or representation thereof and the one or more images of the plurality of second detectable arrays or representations thereof.

The diagnosis can be expressed in quantitative or qualitative terms. For example, a diagnosis of a particular disease, disorder, or condition state can be expressed as a percent similarity to the one or more images of the plurality of second detectable arrays or representations thereof associated with subjects, in-vitro samples, or environmental samples having the particular disease, disorder or condition state. Alternatively, a diagnosis can be expressed as a qualitative likelihood that a subject, in-vitro sample, or environmental sample has, or does not have, a particular disease, disorder, or condition state.

A method of diagnosing a disease, disorder, or condition state in a subject, in-vitro sample, or environmental sample can comprise contacting a first detectable array, such as any of the detectable arrays described herein, with one or more analytes associated with the subject, in-vitro sample, or environmental sample. The first detectable array can be contacted, for example, with one or more body samples of a subject. The first detectable array can be contacted, for example, with at least one of blood, serum, plasma, urine, stool, saliva, bile, spinal fluid, interstitial fluid, gastric juice, tears, solvent, and milk of the subject, in-vitro sample, or environmental sample, such as the plasma, serum, or urine of a subject.

The method of diagnosing can also comprise obtaining the one or more analytes associated with the subject, in-vitro sample, or environmental sample, although this is not required unless otherwise specified. For example, the analytes can be obtained by a third-party, such as a phlebotomist, testing laboratory, or collector of environmental samples, who does not perform the other diagnosing steps. Alternatively the analytes can be obtained by the same entity that conducts the remaining diagnosing steps.

The method of diagnosing can also comprise detecting the one or more analytes associated with the subject, in-vitro sample, or environmental sample, although this is not required unless otherwise specified. For example, detecting can be performed by the same entity that performs the other diagnostic steps, or a different entity. In the latter case, the detectable array, after being contacted with one or more analytes, can be transported to a third party for detection. Detecting the one or more analytes can comprise any of the detection methods discussed herein, for example, one or more of Maillard reaction, caramelizing, reaction with one or more amine reactive dyes, reaction with one or more thiol reactive dyes, reaction with one or more cellular dyes, reaction with one or more solvatochromic dyes, reaction with one or more acid indicators, reaction with one or more base indicators, reaction with one or more labeled antibodies, luminescence, surface texture analysis, photo-scanning, microscopy, photo-scanning with reflectance or transmittance illumination, photography with reflectance or transmittance illumination, mass spectrometry and spectroscopy.

The likelihood of a particular subject, in-vitro sample, or environmental sample to have a disease, disorder, or condition can be determined by comparing features of the image of the first detectable array or representations thereof to corresponding features of the images of the plurality of second detectable arrays or representations thereof in the database. When the images of the plurality of second detectable arrays or representations thereof are associated with the known disease, disorder, or condition of the subject, in-vitro sample, or environmental sample associated with the plurality of second detectable arrays or representations thereof, similarities and differences between the image of the first detectable array or representations and the plurality of second detectable arrays or representations thereof can be used to diagnose the subject, in-vitro sample, or environmental sample associated with the first detectable array.

The detection method can also comprise making one or more images of the detectable array, or one or more representations thereof. The image can be a photograph or digital image. A representation thereof can include a false-color image where features such as texture, depth, surface roughness, luminescence intensity, and the like, are represented by colors or other indicia.

A method of making a detectable array, such as the detectable arrays described herein, can comprise independently coating a plurality of surfaces of a substrate with a least one substrate coating to form a plurality of independently coated surfaces and independently affixing at least one macromolecule or one or more precursors thereof to each of the independently coated surfaces.

The one or more substrate coatings can, independently, coat all or a portion of each of the plurality of surfaces. The substrate coatings can be any coatings that contain appropriate chemical groups for fixing one or more macromolecules to the surface of the substrate. Thus, the identity of the substrate coatings will depend on the identity of the one or more macromolecules to be affixed to the substrate. For example, if the one or more macromolecules include nanotubes, then the substrate coating can have a chemical moiety that binds to nanotubes. As another example, if the one or more macromolecules includes polymer, then the substrate coating can have a functional group that can polymerize into the polymer backbone. Thus, the substrate coating can include, for example, at least one silane or at least one siloxane. Particular siloxanes include one or more acrylosiloxanes, such as one or more of 3-methacryloxypropyl trimethoxy silane, 3-acryloxypropyl trimethoxy silane, N-(3-acryloxy-2-hydroxypropyl-3-aminopropyltriethoxysilane, and 3-methacryloxy propyldimethylchlorosilane.

The macromolecules can be, for example, at least one of polymer, surfactant, nanosphere, nanotube, dendrimer, microsphere, and polymerized microsphere. When the macromolecules include polymer, the polymer can be a homopolymer or copolymer, but is typically a copolymer. The macromolecules can comprise one or more of (meth)acrylamides, (meth)acrylates, and N,N'-(alkylene)bisacrylamide. For example, the macromolecules can comprise one or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-tert-octylacrylamide. As another example, the macromolecules can comprise two or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-tert-octylacrylamide.

The macromolecules can also comprise at least one cross-linker. The cross-linker can be any cross-linker known in the art. Cross-linkers can include, for example, one or more molecules containing two, three, four, or more olefins or acrylic functional groups, such as one or more of bis-acrylamide, trimethylolpropane triacrylate, bisphenol A-bis(2-hydrxypropyl)acrylate, and 1-(acryloyloxy)-3-(methacryloyloxy)-3-methacryloyloxy)-2-propanol.

The macromolecule precursor can be, for example one or more monomers. The one or more monomers can comprise one or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-tert-octylacrylamide. As another example, the macromolecules can comprise two or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-diemthyl-3-oxobutyl)acrylamide, N-isopropylacrylamide, ethylene glycol phenyl ether acrylate, N,N'-methylenebisacrylamide, 1,1,3,3,3-hexafluoroisopropyl acrylate, and N-tert-octylacrylamide.

The method can further comprise polymerizing one or more precursors of macromolecules, such as monomers and particularly olefin containing monomers, on at least one of the independently coated surfaces of the substrate. The polymerization can be initiated by any known method of initiating polymerizations. For example, the polymerization can be initiated by applying one or more of light, heat, or a chemical initiator to the one or more precursors. The light can be any light that is capable of initiating the polymerization, such as visible light, ultraviolet light, ionizing radiation, and the like. The heat can be any temperature that is capable of initiating the polymerization; such temperatures will be known or readily determinable by a person of skill in the art. Chemical initiators are known in the art, and can be used in conjunction with light or heat if needed. Typical chemical initiators include azo and peroxy compounds, such as dicumyl peroxide and AIBN, persulfates, and the like. In another embodiment, the method comprises initiating polymerization of at least one of the one or more precursors by applying one or more of electromagnetic radiation (*e.g.,* microwaves, ultraviolet light, visible light, heat), sound or a chemical initiator to the one or more precursors.

Importantly, while specific types of arrays are described herein, other arrays can also be used unless otherwise specified. This is particularly true when unlabeled analytes are used. Thus, a method of detecting an unlabeled analyte can include contacting the unlabeled analyte with any detectable array, including those discussed herein and others, and heating the detectable array with the unlabeled analyte affixed thereto to cause a color change of at least one of the unlabeled analyte and the detectable array.

The contacting step can be sufficient to affix at least a portion of the analyte to the detectable array. The analyte can be any analyte, such as one or more of a body fluid of a subject, in-vitro sample, environmental sample, and a component of one or more of the foregoing. For example, the analyte can be a component of least one of blood, serum, plasma, urine, stool, saliva, bile, spinal fluid, interstitial fluid, gastric juice, tears, solvent, and milk.

The color change can relate to non-enzymatic browning of the analyte. The non-enzymatic browning can be any form of non-enzymatic browning, for example, one or more of a Maillard reaction and caramelization. The heating step can be for a sufficient time and at a sufficient temperature to induce the Maillard reaction, caramelization, or both. Such temperatures and times are discussed herein, and can be, for example, temperatures of about 120° C to about 300° C for about 1 minute to about 5 minutes. Once the color change has occurred, the detectable array can be analyzed by any known methods, including but not limited to the methods discussed herein.

This method can be used with any type of detectable array, not only the detectable arrays discussed in detail herein. For example, the method can be used with one or more of an analytical microarray, a reverse-phase micro assay, a functional microarray, a cell-containing microarray, an expression microarray, and a high-throughput array. As another example, the method can be used with one or more arrays selected from the group consisting of an antibody array, and ELISA array, a peptide array, a protein array, a nucleotide array, a nucleoside array, an RNA array, a DNA array, a DNA-protein array, and a small molecule array.

### EXAMPLES

### Example 1 - Substrate Coating

A borosilicate slide is cleaned with absolute ethanol and allowed to dry. 200 µL of a silanization solution having the components of Table 1 is applied to one side of the slide and allowed to spread over the entire side. The side is wiped with a sterile paper wipe to remove excess solution, and then placed on a slide holder with the coated surface facing a heat source at 150° C for about 30 minutes. The slide is then cooled to room temperature, and then washed in fresh absolute ethanol. Slides are dried by blowing filtered air (0.02 µm filter) over the slides.

**Table 1**

| **Component** | **Amount (vol. %)** |
|---|---|
| Absolute ethanol | 94.5 |
| Distilled water | 5 |
| Acetic acid (neat) | 0.5 |
| 3-methacryloxypropyl trimethoxysilane | 0.3 |

### Example 2 - Macromolecule Precursors

Stock solutions of monomers were prepared according to Table 2. All of the monomers were obtained commercially except for histamine acrylate, which was prepared by mixing an equimolar amount of histamine and acrylic acid in a solution of phosphate buffered saline (PBS) and dimethyl sulfoxide (DMSO).

**Table 2**

| **Solution Number** | **Monomer Name** | **Monomer Dilution** | **Acrylamide** | **Solvent** | **Concentration of Monomers** |
|---|---|---|---|---|---|
| 1 | 2-Carboxyethyl acrylate | 482 uL / mL = 4M | None | DMSO | 4M |
| 2 | Acrylic Acid | 288 uL / mL = 4M | None | DMSO | 4M |
| 3 | Histamine acrylate | 2M | 50 mg / 250 uL 2M histamine acrylate | 50:50 DMSO:PBS | 1.67 M monomer/ 2.3 M acrylamide |
| 4 | N-[tris(hydroxymethyl)methy] acrylamide | 350.4 mg / mL = 2M | 1:1 2M monomer: 6M acrylamide | 90% glycerol + 10% glycerol | 1M monomer/3 M acrylamide |
| 5 | Hydroxypropyl acrylate, isomers | 498.63 uL / mL = 4M | None | DMSO | 4M |
| 6 | 4-hydroxybutyl acrylate | 576.7 uL / mL = 4M | None | DMSO | 4M |
| 7 | N-Hydroxyethyl acrylamide (Polysciences, Inc., #25109) | 414.9 uL / mL = 4M | None | DMSO | 4M |
| 8 | N,N-Dimethylacrylamide | 412.19 uL / mL = 4M | None | DMSO | 4M |
| 9 | N-(1,1-Dimethyl-3-oxobutyl) acrylamide | 676.9 mg / mL = 4M | 1:1 4M monomer: 4M acrylamide | DMSO | 2 M monomer/2 M acrylamide |
| 10 | N-iso-propylacrylamide | 452.8 mg / mL = 4M | None | DMSO | 4M |
| 11 | Ethylene glycol phenyl ether acrylate (Santa Cruz Biotech, 239963) | 696.4 uL / mL = 4M | 1:5 4M monomer: 4M acrylamide | DMSO | 0.67 M monomer/ 3.33M acrylamide |
| 12 | N-Tert-Octylacrylamide | 733.2 mg / mL = 4M | 1:5 4M monomer : 4M acrylamide | DMSO | 0.67 M monomer/3.33 M acrylamide |

### Example 3 - Monomer Mixtures

Each of the twelve monomer solutions from Table 2 in Example 2 were chilled. Twelve vessels are each charged with three volumes of the polymerization mixture of Table 3 are added to the chilled monomer solutions. One volume of the one of the solutions 1-12 from Table 2 is added to each of the twelve vessels to make monomer mixtures 1-12, each of which contains both the monomers of the corresponding solution 1-12 of Table 2 and the materials of Table 3

**Table 3**

| **Reagent** | **Concentration** **in Solvent** | **Final** **Concentration** **(when diluted 3:1)** |
|---|---|---|
| **Acrylamide** | 0.67 M | 0.5 M |
| **Bis-Acrylamide** (as 31 mg/mL DMSO solution) | 49.9 mM (0.33%) | 37.5 mM (0.25%) |
| **DMPA** (as 800 mM DMSO solution) | 33.3 mM | 25 mM |
| **Glycerol** | 33.25% | 25% |
| **DMSO** | q.s to 100% | |

### Example 4 - Master Plates

Equal volumes of each of the monomer mixtures 1 to 12 were added to wells of a 96-well plate according to Table 4, making mixtures of the various monomers. The numbers in the table correspond to the identity of the monomer stock solutions (from Table 2) that are present in each well.

**Table 4**

| | 1 | 2 | 3 | 4 | 5 | 6 | 7 | 8 | 9 | 10 | 11 | 12 |
|---|---|---|---|---|---|---|---|---|---|---|---|---|
| 1 | 1-1 | 1-2 | 1-3 | 1-4 | 1-5 | 1-6 | 1-7 | 1-8 | 1-9 | 1-10 | 1-11 | 1-12 |
| 2 | | 2-2 | 2-3 | 2-4 | 2-5 | 2-6 | 2-7 | 2-8 | 2-9 | 2-10 | 2-11 | 2-12 |
| 3 | | | 3-3 | 3-4 | 3-5 | 3-6 | 3-7 | 3-8 | 3-9 | 3-10 | 3-11 | 3-12 |
| 4 | | | | 4-4 | 4-5 | 4-6 | 4-7 | 4-8 | 4-9 | 4-10 | 4-11 | 4-12 |
| 5 | | | | | 5-5 | 5-6 | 5-7 | 5-8 | 5-9 | 5-10 | 5-11 | 5-12 |
| 6 | | | | | | 6-6 | 6-7 | 6-8 | 6-9 | 6-10 | 6-11 | 6-12 |
| 7 | | | | | | | 7-7 | 7-8 | 7-9 | 7-10 | 7-11 | 7-12 |
| 8 | | | | | | | | 8-8 | 8-9 | 8-10 | 8-11 | 8-12 |

The mixtures are of Table 4 were then transferred to a second 96-well plate in the format of Table 5. Again, the numbers in the table correspond to the identity of the monomer stock solutions (from Table 2) that are present in each well.

**Table 5**

| | 1 | 2 | 3 | 4 | 5 | 6 | 7 | 8 | 9 | 10 | 11 | 12 |
|---|---|---|---|---|---|---|---|---|---|---|---|---|
| 1 | 1-1 | 1-2 | 1-3 | 1-4 | 1-5 | 1-6 | 1-7 | 1-8 | 1-9 | 1-10 | 1-11 | 1-12 |
| 2 | 8-8 | 2-2 | 2-3 | 2-4 | 2-5 | 2-6 | 2-7 | 2-8 | 2-9 | 2-10 | 2-11 | 2-12 |
| 3 | 8-9 | 9-9 | 3-3 | 3-4 | 3-5 | 3-6 | 3-7 | 3-8 | 3-9 | 3-10 | 3-11 | 3-12 |
| 4 | 8-10 | 10-10 | 7-7 | 4-4 | 4-5 | 4-6 | 4-7 | 4-8 | 4-9 | 4-10 | 4-11 | 4-12 |
| 5 | 8-11 | 11-11 | 7-8 | 7-10 | 5-5 | 5-6 | 5-7 | 5-8 | 5-9 | 5-10 | 5-11 | 5-12 |
| 6 | 8-12 | 12-12 | 7-9 | 7-11 | 7-12 | 6-6 | 6-7 | 6-8 | 6-9 | 6-10 | 6-11 | 6-12 |
| 7 | | | | | | | | | | | | |
| 8 | | | | | | | | | | | | |

30 µL of each of the charged wells of Table 5 are transferred to a 384-well plate in the format of Table 6. The 384-well plate is kept chilled on a cold surface to minimize evaporation.

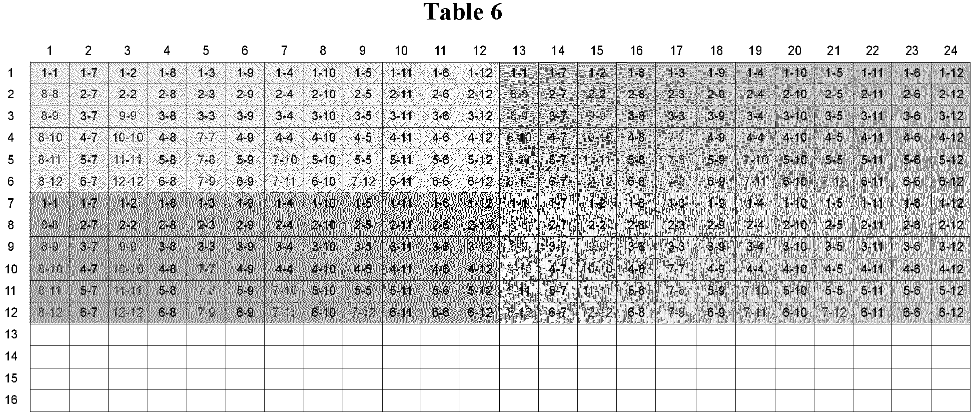

The 384-well plate in Table 6 has four quadrants, each having 72 monomer mixtures. The four quadrants, I-IV, are (in row-column format) 1-1 to 6-12, 1-13 to 6-24, 1-7 to 12-12, and 13-7 to 12-24. 10 µL of one of monomer mixtures 1 to 4 (from monomer mixtures 1 to 2, Example 3) is added to each well of quadrants I-IV, respectively, thus providing 288 wells with chemically unique compositions. The plates were chilled and covered with a plate cover or parafilm to minimize evaporation until use.

Two additional 384-well plates are prepared, each of which is identical to the one described above except for the identity of the 10 µL of monomer mixture added. The second plate has 10 µL of monomer mixtures 5 to 8 added to quadrants I-IV, respectively, and the third has 10 µL of monomer mixtures 9 to 12 added to quadrants I-IV, respectively. The resulting three plates each have 288 different combinations of polymer precursors.

### Example 6 - Detectable Array

The combinations of polymer precursors in the three plates prepared according to Example 5 are coated onto a substrate prepared according to Example 1 by applying each of the different combinations of polymer precursors to a distinct area of the substrate. The polymer precursors are then polymerized by application of ultraviolet light. The functionalized silane on the substrate coating is incorporated into the polymer backbones, thereby affixing the polymers to the substrate.

### Example 7 - System

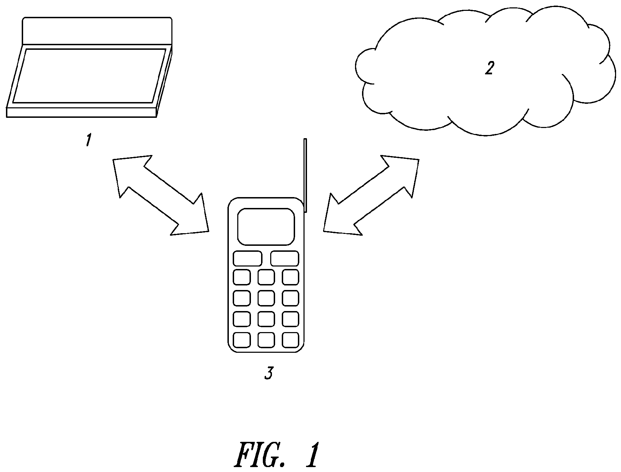

Turning to the figures, a schematic of an exemplary system is shown in Figure 1. The system includes a photo-scanner **1** for receiving one or more images of a first detectable array or representations thereof. The photo-scanner **1** can also convert the one or more images of a first detectable array or representations thereof into one or more digital file, such as a jpeg file, a pdf file, a tiff file, a gif file, or other type of file that contains information from the one or more images. Importantly, while the input element in Figure 1 is photo-scanner **1,** which can both create and receive one or more images or representations thereof, other input elements, including those that can only receive one or more images or representations thereof, can also be used.

The system also includes a cloud-computing database **2,** which is stored on one or more computer servers in a cloud-computing environment. Importantly, other types of databases can also be used. The cloud-computing database **2** comprises digital image files of a plurality of second detectable arrays, each of which was exposed to human plasma and detected for the presence of one or more analytes bound to the surface. Each digital image file is associated with a human patient having at least one known disease, disorder, or condition (with the understanding that good health or lack of disease can be a known condition), and contains, in addition to the digital image, information regarding the at least one known disease, disorder, or condition. In this example, the cloud-computing database **2** also comprises software for receiving a digital file one or more images of the first detectable array or representations thereof from the photo-scanner **1,** and for comparing the one or more images of the first detectable array, or representations thereof, such as the one or more digital files, to the digital image files of the plurality of second detectable arrays. Importantly, in addition or in the alternative to being part of the database element, such software could be located in the comparison element.

A smart-phone **3** is also included in the system. In this example, the smart phone 3 is a comparison element that is adapted to connect with cloud-computing database **2,** and compares the one or more digital image files of the first array with the plurality of image files of the plurality of second arrays by either executing or causing to be executed the software, located in the cloud-computing database **2,** for comparing the one or more images of the first detectable array, or representations thereof to the digital image files of the plurality of second detectable arrays. In this example, the smart-phone **3** is also adapted to receive the results of the comparison in the form of a list of diseases or disorders and an estimation of the likelihood that the subject has or is susceptible to the each of diseases or disorders on the list.

### Example 8 - Detecting Proteins (according to the claimed invention)

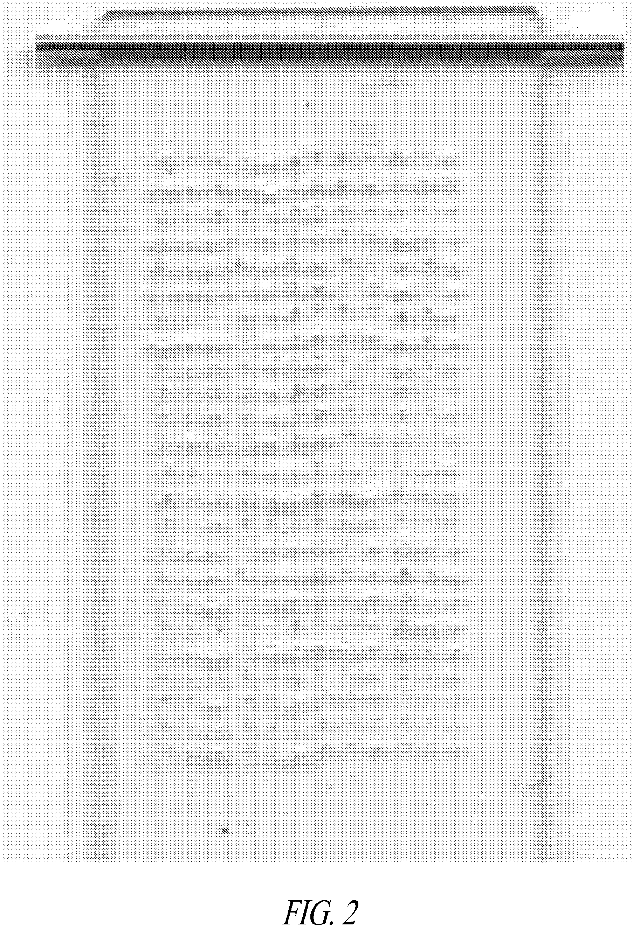

A detectable array of Example 6 was washed with distilled water for 15 minutes and air dried. A serum sample was contacted with the detectable array such that all of the surfaces of the detectable array contacted the serum sample. The serum sample was allowed to remain on the detectable array for 15 minutes at ambient temperature, and then removed by shaking the array. The array was washed by immersion in distilled water for about 5 seconds, followed by removal of excess water by shaking the array. The array was then heated for 5 minutes at 300° C to induce Maillard reactions between serum proteins and other molecules bound to the array. After cooling to room temperature, the array was scanned using a commercial photo-scanner at 1,000 dpi resolution. An image of the scanned array appears in Figure 2. This example shows that serum proteins can be bound to and detected on the detectable array.

### Example 9 - Detecting Cells

Yeast cells were removed from a culture by diluting with phosphate buffered saline (PBS) at pH 7.4 and pelleted by centrifugation. The cells were resuspended in PBS and 10 µL of 3-(4,5-dimethylthiazol-2-yl)-2,5-diphenyltetrazolium bromide (MTT) was added per 100,000 cells. The cells were incubated in the MTT at 37° C for about one hour. The cells were pelleted by centrifugation and re-suspended in PBS.

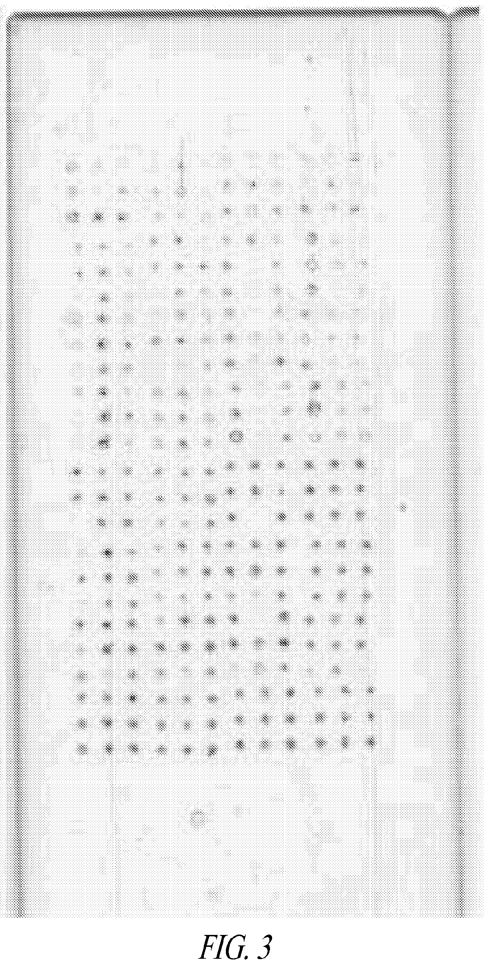

A detectable array of Example 6 was washed in distilled water for 15 minutes at ambient temperature and air dried. The cell suspension was contacted to the array such that all of the surfaces of the detectable array contacted the suspension. The array was incubated with the suspension for 30 minutes at ambient temperature with mild agitation. The array was dried by shaking off the excess suspension. The array was then washed by immersion in distilled water for about 5 seconds, followed by removal of excess water by shaking the array. The array was then scanned using a commercial photo-scanner at 1,000 dpi resolution. An image of the scanned array appears in Figure 3. This example shows that cells can be bound to and detected on the detectable array.

### Example 10 - Detecting Small Molecules (according to the claimed invention)

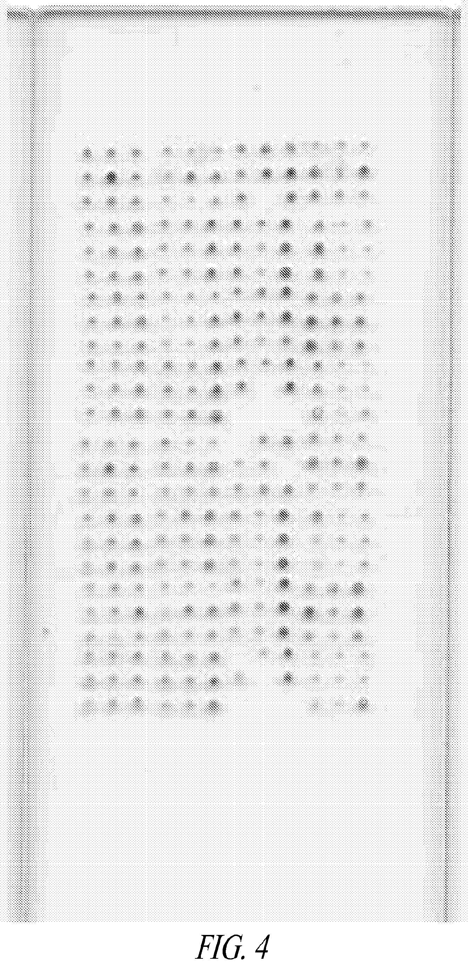

A detectable array of Example 6 was washed in distilled water for 15 minutes at ambient temperature and air dried. A sample of sucralose, dextrose, and maltodextrin sold under the name SPLENDA® was dissolved in distilled water and the solution contacted with the array such that all of the surfaces of the detectable array contacted the solution. The solution was allowed to remain on the detectable array for 15 minutes at ambient temperature, after which excess solution was removed by shaking the array. he array was washed by immersion in distilled water for about 5 seconds, followed by removal of excess water by shaking the array. The array was then heated for 5 minutes at 300° C to induce Maillard reactions between serum proteins and other molecules bound to the array. After cooling to room temperature, the array was scanned using a commercial photo-scanner at 1,000 dpi resolution. An image of the scanned array appears in Figure 4. This example shows that small molecules can be bound to and detected on the detectable array.

### Example 11 - Detecting Commercial Arrays

A commercial array with protein ligands is obtained from a commercial supplier. A plasma sample is contacted with the commercial array such that one or more of the plasma proteins bind to the ligands. The array is then washed to remove any unbound materials.

The proteins are detected by heating the array in a commercial oven to 300° C for about 5 minutes to induce a Maillard reaction. The array turns brown at the locations having ligands with bound plasma protein; this color change allows for the identification of which ligands the plasma proteins are bound to.
The disclosed embodiments use specific examples and descriptive language to allow a person of skill in the art to make, use, and practice the invention. However, it should be understood that the disclosure is not meant to be limiting. Thus, the scope of protection is determined by the appended claims. In particular, a person of skill in the art will recognize variations of the disclosed embodiments which fall within the scope of the appended claims. Thus, the invention is not to be limited to the embodiments discussed herein. For example, while particular analytes and methods of detecting those analytes are discussed for illustrative purposes, other analytes and detection methods can also be used.

## Claims

1. A label-free method of detecting an unlabeled analyte on a detectable array,
said detectable array comprising:
a substrate with a plurality of surfaces for binding one or more analytes, each surface independently comprising:
one or more substrate coatings on the surface for fixing one or more macromolecules to the surface of the substrate, said one or more substrate coatings comprising at least one silane or at least one siloxane; and
one or more macromolecules affixed to at least a portion of the one or more substrate coatings, the one or more macromolecules being arranged in a pattern on the substrate coating and comprising a plurality of binding sites for binding a plurality of analytes;
wherein each macromolecule has been affixed to the substrate coating by polymerizing a functional group on the silane or siloxane into the macromolecule backbone;
wherein each macromolecule comprises a polymer of monomers selected from a group consisting of acrylamide; 2-carboxyethyl acrylate; acrylic acid; N-[tris(hydroxymethyl)methy] acrylamide; hydroxypropyl acrylates; 4-hydroxybutyl acrylate; N-hydroxyethyl acrylamide; N,N-dimethylacrylamide; N-(1,1-dimethyl-3-oxobutyl) acrylamide; N-iso-propylacrylamide; (meth)acrylates; 2-cyanoethyl acrylate; ethylene glycol phenyl ether acrylate; N-tert-octylacrylamide; 1-(acryloyloxy)-3-(methacryloyloxy)-2-propanol; bis-acrylamide; trimethylolpropane triacrylate, and combinations thereof; and
wherein the identity, pattern, or both identity and pattern of the one or more macromolecules on the one or more substrate coatings of each of the plurality of surfaces is not identical to the identity, pattern, or both identity and pattern of the one or more macromolecules on any other substrate coating of any other of the plurality of surfaces;
said label-free method comprising:
contacting the unlabeled analyte with the detectable array to affix at least a portion of the analyte to the detectable array; and
heating the detectable array with the unlabeled analyte affixed thereto to cause a color change of at least one of the analyte and the detectable array, wherein the heating induces a non-enzymatic browning reaction, and wherein the heating comprises heating at a temperature of about 120°C to about 300°C for about 1 minute to about 5 minutes; and
assessing the color of each of the plurality of surfaces of the substrate by colorimetric analytical technique.

2. The method of claim 1, wherein the non-enzymatic browning reaction is at least one of a Maillard reaction and caramelization.

3. The method of claim 1 or 2,
wherein the one or more substrate coatings comprise one or more acrylosiloxanes.

4. The method of any of claims 1-3,
wherein the one or more substrate coatings comprise one or more of 3-methacryloxypropyl trimethoxy silane, 3-acryloxypropyl trimethoxy silane, N-(3-acryloxy-2-hydroxypropyl-3-aminopropyltriethoxysilane, and 3-methacryloxypropyl dimethylchlorosilane.

5. The method of any of claims 1-4,
wherein the substrate comprises at least 2 chemically distinct macromolecules, and wherein each chemically distinct macromolecule is affixed to a different surface of the plurality of surfaces of the substrate, and wherein, optionally, the one or more macromolecules comprise
at least 12 chemically distinct macromolecules,
at least 72 chemically distinct macromolecules,
at least 96 chemically distinct macromolecules, or
at least 288 chemically distinct macromolecules.

6. The method of any of claims 1-5,
wherein the substrate comprises one or more of glass, plastic, metal, composites, acrylics, or biologically active substrates.

7. The method of any of claims 1-6,
wherein the unlabeled analyte is selected from (i) a body sample of a subject, (ii) an *in-vitro* sample, (iii) an environmental sample, and (iv) a component of one of the foregoing.

8. The method of any of claims 1-7,
wherein the unlabeled analyte is at least one component of blood, serum, plasma, urine, stool, saliva, bile, spinal fluid, interstitial fluid, gastric juice, tears, solvent, and milk.

9. The method of any of claims 1-8,
wherein the unlabeled analyte comprises a protein or peptide.

## Patentansprüche

1. Markierungsfreies Verfahren zum Nachweis eines unmarkierten Analyten auf einem nachweisbaren Array, wobei das nachweisbare Array umfasst:
ein Substrat mit einer Vielzahl von Oberflächen zum Binden eines oder mehrerer Analyten, wobei jede Oberfläche unabhängig umfasst:
eine oder mehrere Substratbeschichtungen auf der Oberfläche zum Fixieren eines oder mehrerer Makromoleküle an der Oberfläche des Substrats, wobei die eine oder mehreren Substratbeschichtungen mindestens ein Silan oder mindestens ein Siloxan umfassen; und
ein oder mehrere Makromoleküle, die an mindestens einem Teil der einen oder mehreren Substratbeschichtungen befestigt sind, wobei das eine oder die mehreren Makromoleküle in einem Muster auf der Substratbeschichtung angeordnet sind und eine Vielzahl von Bindungsstellen zum Binden einer Vielzahl von Analyten umfassen;
wobei jedes Makromolekül durch Polymerisieren einer funktionellen Gruppe am Silan oder Siloxan in das Makromolekülgerüst an der Substratbeschichtung befestigt worden ist;
wobei jedes Makromolekül ein Polymer von Monomeren umfasst, ausgewählt aus einer Gruppe bestehend aus Acrylamid; 2-Carboxyethylacrylat; Acrylsäure; N-[Tris(hydroxymethyl)methyl]acrylamid; Hydroxypropylacrylaten; 4-Hydroybutylacrylat; N-Hydroxyethylacrylamid; N,N,-Dimethylacrylamid; N-(1,1-Diemthyl-3-oxobutyl)acrylamid; N-Isopropylacrylamid; (Meth)acrylaten; 2-Cyanoethylacrylat; Ethylenglycolphenyletheracrylat; N-tert-Octylacrylamid; 1-(Acryloyloxy)-3-(methacryloyloxy)-2-propanol; Bisacrylamid; Trimethylolpropantriacrylat und Kombinationen davon; und
wobei die Identität, das Muster oder sowohl die Identität als auch das Muster des einen oder der mehreren Makromoleküle auf der einen oder den mehreren Substratbeschichtungen von jeder der Vielzahl von Oberflächen nicht mit der Identität, dem Muster oder sowohl der Identität als auch dem Muster des einen oder der mehreren Makromoleküle auf jeder anderen Substratbeschichtung von jeder anderen der Vielzahl von Oberflächen identisch ist;
wobei das markierungsfreie Verfahren umfasst:
Inkontaktbringen des unmarkierten Analyten mit dem nachweisbaren Array, um mindestens einen Teil des Analyten an dem nachweisbaren Array zu befestigen; und
Erhitzen des nachweisbaren Arrays mit dem daran befestigten unmarkierten Analyten, um eine Farbänderung von mindestens einem des Analyten und des nachweisbaren Arrays zu bewirken, wobei das Erhitzen eine nichtenzymatische Bräunungsreaktion induziert und wobei das Erhitzen das Erhitzen auf eine Temperatur von ungefähr 120°C bis ungefähr 300°C für ungefähr 1 Minute bis ungefähr 5 Minuten umfasst; und
Beurteilen der Farbe jeder der Vielzahl von Oberflächen des Substrats durch colorimetrische Analysetechnik.

2. Verfahren nach Anspruch 1, wobei die nichtenzymatische Bräunungsreaktion mindestens eine Maillard-Reaktion oder Karamellisierung ist.

3. Verfahren nach Anspruch 1 oder 2, wobei die eine oder mehreren Substratbeschichtungen ein oder mehrere Acrylosiloxane umfassen.

4. Verfahren nach einem der Ansprüche 1-3, wobei die eine oder mehreren Substratbeschichtungen ein oder mehrere von 3-Methacryloxypropyltrimethoxysilan, 3-Acryloxypropyltrimethoxysilan, N-(3-Acryloxy-2-hydroxypropyl-3-aminopropyltriethoxysilan und 3-Methacryloxypropyldimethylchlorsilan umfassen.

5. Verfahren nach einem der Ansprüche 1-4, wobei das Substrat mindestens 2 chemisch unterschiedliche Makromoleküle umfasst und wobei jedes der chemisch unterschiedlichen Makromoleküle an einer anderen Oberfläche der Vielzahl von Oberflächen des Substrats befestigt ist und wobei wahlweise das eine oder die mehreren Makromoleküle mindestens 12 chemisch unterschiedliche Makromoleküle, mindestens 78 chemisch unterschiedliche Makromoleküle, mindestens 96 chemisch unterschiedliche Makromoleküle oder mindestens 288 chemisch unterschiedliche Makromoleküle umfassen.

6. Verfahren nach einem der Ansprüche 1-5, wobei das Substrat ein oder mehr von Glas, Kunststoff, Metall, Verbundwerkstoffen, Acryl oder biologisch aktiven Substraten umfasst.

7. Verfahren nach einem der Ansprüche 1-6, wobei der unmarkierte Analyt aus (i) einer Körperprobe eines Subjekts, (ii) einer *In-vitro*-Probe, (iii) einer Umweltprobe und (iv) einer Komponente einer der vorgenannten ausgewählt ist.

8. Verfahren nach einem der Ansprüche 1-7, wobei der unmarkierte Analyt mindestens eine Komponente von Blut, Serum, Plasma, Urin, Stuhl, Speichel, Galle, Rückenmarksflüssigkeit, interstitieller Flüssigkeit, Magensaft, Tränen, Lösungsmittel und Milch ist.

9. Verfahren nach einem der Ansprüche 1-8, wobei der unmarkierte Analyt ein Protein oder ein Peptid umfasst.

## Revendications

1. Procédé sans étiquette pour détecter un analyte non marqué sur un réseau détectable,
ledit réseau détectable comprenant:
un substrat avec une pluralité de surfaces pour la liaison d'un ou plusieurs analytes, chaque surface comprenant indépendamment:
un ou plusieurs revêtements de substrat sur la surface pour fixer une ou plusieurs macromolécules à la surface du substrat, lesdits un ou plusieurs revêtements de substrat comprenant au moins un silane ou au moins un siloxane; et
une ou plusieurs macromolécules fixées à au moins une partie du ou des revêtements de substrat, la ou les macromolécules étant disposées selon un motif sur le revêtement de substrat et comprenant une pluralité de sites de liaison pour lier une pluralité d'analytes;
dans lequel chaque macromolécule a été fixée au revêtement du substrat par polymérisation d'un groupe fonctionnel sur le silane ou le siloxane dans le squelette de la macromolécule;
dans lequel chaque macromolécule comprend un polymère de mono-mères choisis dans le groupe constitué par l'acrylamide, l'acrylate de 2-carboxyéthyle, l'acide acrylique, le N-[tris(hydroxyméthyl)méthyl] acrylamide, les acrylates d'hydro-xypropyle, l'acrylate de 4-hydroxybutyle, le N-hydroxyéthyl acrylamide, le N,N-diméthylacrylamide; le N-(1,1-diméthyl-3-oxobutyl) acrylamide; le N-iso-propyl-acrylamide; (méth)acrylates; l'acrylate de 2-cyanoéthyle; l'acrylate d'éther phénylique d'éthylène glycol; le N-tert-octylacrylamide; le 1-(acryloyloxy)-3-(méthacryloyloxy)-2- propanol; le bis-acrylamide; le triacrylate de triméthylolpropane, et leurs combinaisons; et
dans lequel l'identité, le motif ou à la fois l'identité et le motif de la ou des macromolécules sur le ou les revêtements de substrat de chacune des surfaces de la pluralité de surfaces n'est pas identique à l'identité, au motif ou à la fois l'identité et le motif de la ou des macromolécules sur tout autre revêtement de substrat de toute autre surface de la pluralité de surfaces;
ledit procédé sans label comprenant:
la mise en contact de l'analyte non marqué avec le réseau détectable afin de fixer au moins une partie de l'analyte au réseau détectable; et
le chauffage du réseau détectable avec l'analyte non marqué qui y est fixé pour provoquer un changement de couleur d'au moins l'un de l'analyte et du réseau détectable, dans lequel le chauffage induit une réaction de brunissement non enzyma-tique, et dans lequel le chauffage comprend le chauffage à une température d'environ 120°C à environ 300°C pendant environ 1 minute à environ 5 minutes; et
l'évaluation de la couleur de chacune des surfaces de la pluralité des surfaces du substrat par une technique analytique colorimétrique.

2. Procédé selon la revendication 1, dans lequel la réaction de brunissement non enzymatique est au moins une réaction de Maillard et de caramélisation.

3. Procédé selon les revendications 1 ou 2,
dans lequel le ou les revêtements de substrat comprennent un ou plusieurs acrylo-siloxanes.

4. Procédé selon l'une des revendications 1 à 3,
dans lequel le ou les revêtements de substrat comprennent un ou plusieurs des produits suivants: 3-méthacryloxypropyl triméthoxysilane, 3-acryloxypropyl triméthoxysilane, N-(3-acryloxy-2-hydroxypropyl-3- aminopropyltri éthoxysilane et 3-méthacryloxy-propyl diméthylchlorosilane.

5. Procédé selon l'une des revendications 1 à 4,
dans lequel le substrat comprend au moins 2 macromolécules chimiquement distinctes, et dans lequel chaque macromolécule chimiquement distincte est fixée à une surface différente de la pluralité de surfaces du substrat, et dans lequel, éventuellement, la ou les macromolécules comprennent:
au moins 12 macromolécules chimiquement distinctes,
au moins 72 macromolécules chimiquement distinctes,
au moins 96 macromolécules chimiquement distinctes, ou
au moins 288 macromolécules chimiquement distinctes.

6. Procédé selon l'une des revendications 1 à 5,
dans lequel le substrat comprend un ou plusieurs des matériaux suivants : verre, plastique, métal, composites, acryliques ou substrats biologiquement actifs.

7. Procédé selon l'une des revendications 1 à 6,
dans lequel l'analyte non marqué est choisi parmi (i) un échantillon de corps d'un sujet, (ii) un échantillon *in vitro,* (iii) un échantillon environnemental, et (iv) un composant de l'un des éléments précédents.

8. Procédé selon l'une des revendications 1 à 7,
dans lequel l'analyte non marqué est au moins un composant du sang, du sérum, du plasma, de l'urine, des selles, de la salive, de la bile, du liquide céphalo-rachidien, du liquide interstitiel, du suc gastrique, des larmes, du solvant et du lait.

9. Procédé selon l'une des revendications 1 à 8,
dans lequel l'analyte non marqué comprend une protéine ou un peptide.
